# FASCICULE DE BREVET EUROPEEN

(11) **EP 2 935 212 B1**
(45) Date de publication et mention de la délivrance du brevet: **27.02.2019**
(21) Numéro de dépôt: 13814948.9
(22) Date de dépôt: 20.12.2013
(51) Int. Cl.: C07D 211/16, C07D 211/18, C07D 211/22, C07D 295/185, C07D 401/04, C07D 207/08, A61K 31/451, A61K 31/495, A61P 31/04

(54) **HÉTÉROCYCLES AZOTÉS SATURÉS ET N-ACYLÉS POTENTIALISANT L'ACTIVITÉ D'UN ANTIBIOTIQUE ACTIF CONTRE LES MYCOBACTÉRIES**
GESÄTTIGTE UND N-ACYLIERTE STICKSTOFF-HETEROZYKLEN DIE DIE WIRKUNG EINES GEGEN MYKOBACTERIEN AKTIVEN ANTIBIOTIKUMS VERSTÄRKEN
SATURATED AND N-ACYLATED NITROGEN HETEROCYCLES POTENTIATING THE ACTIVITY OF AN ANTIBIOTIC ACTIVE AGAINST MYCOBACTERIA

(30) Priorité: 21.12.2012 FR 1203548
(43) Date de publication de la demande: 28.10.2015
(73) Titulaire: Université de Droit et de la Santé de Lille 2, 59800 Lille (FR)
(72) Inventeur: WILLAND, Nicolas, F-59000 Lille (FR); DEPREZ, Benoit, F-59000 Lille (FR); BAULARD, Alain, B-7520 Tournai (BE); BRODIN, Priscille, F-75014 Paris (FR); FLIPO, Marion, F-5900 Lille (FR); MAINGOT, Lucie, Cambridge Cambridgeshire CB1 7AA (GB)
(74) Mandataire: Latscha Schöllhorn Partner AG
(86) Numéro de dépôt international: PCT/EP2013/077732
(87) Numéro de publication internationale: WO 2014/096378

(56) Documents cités:
- WO-A1-2008/003861
- WO-A1-2010/005783
- WO-A1-2010/063774
- WO-A2-2010/044885
- TSUTOMU IRIKURA ET AL: "New Analgetic Agents. V. 1-Butyryl-4-cinnamylpiperazine Hydrochloride and Related Compounds", JOURNAL OF MEDICINAL CHEMISTRY, vol. 11, no. 4, 1968, pages 801-804, XP002930057, ISSN: 0022-2623, DOI: 10.1021/JM00310A022
- ZHANJIN ZHANG ET AL: "Highly efficient and practical phosphoramidite-copper catalysts for amination of aryl iodides and heteroaryl bromides with alkylamines and N(H)-heterocycles", TETRAHEDRON, vol. 62, no. 18, 2006, pages 4435-4443, XP025001882, ISSN: 0040-4020, DOI: 10.1016/J.TET.2006.02.062 [extrait le 2006-05-01]
- GUIYING LI ET AL: "Design and synthesis of 4-arylpiperidinyl amide and N-arylpiperidin-3-yl-cyclopropane carboxamide derivatives as novel melatonin receptor ligands", BIOORGANIC & MEDICINAL CHEMISTRY LETTERS, vol. 21, no. 4, 19 octobre 2010 (2010-10-19), pages 1236-1242, XP028138677, ISSN: 0960-894X, DOI: 10.1016/J.BMCL.2010.12.068 [extrait le 2010-12-19]
- MARION FLIPO ET AL: "Ethionamide Boosters: Synthesis, Biological Activity, and Structure-Activity Relationships of a Series of 1,2,4-Oxadiazole EthR Inhibitors", JOURNAL OF MEDICINAL CHEMISTRY, vol. 54, no. 8, 2011, pages 2994-3010, XP055019892, ISSN: 0022-2623, DOI: 10.1021/jm200076a cité dans la demande
- MARION FLIPO ET AL: "Ethionamide Boosters. 2. Combining Bioisosteric Replacement and Structure-Based Drug Design To Solve Pharmacokinetic Issues in a Series of Potent 1,2,4-Oxadiazole EthR Inhibitors", JOURNAL OF MEDICINAL CHEMISTRY, vol. 55, no. 1, 12 janvier 2012 (2012-01-12), pages 68-83, XP055068481, ISSN: 0022-2623, DOI: 10.1021/jm200825u

## Description

La présente invention concerne un composé pour son utilisation dans le traitement des infections bactériennes et mycobactériennes comme, par exemple, la tuberculose, la lèpre et les infections mycobactériennes atypiques.

La présente invention concerne également de nouveaux composés utilisables en tant que médicament en particulier en tant que médicament pour le traitement d'infections bactériennes et mycobactériennes comme, par exemple, la tuberculose, la lèpre et les infections mycobactériennes atypiques.

La présente invention concerne également des compositions pharmaceutiques comprenant, en tant que principe actif, au moins un des composés précités et éventuellement un antibiotique actif contre les bactéries et/ou mycobactéries, en particulier un antibiotique activable selon la voie de l'EthA, plus particulièrement un antibiotique choisi parmi la famille des thioamides, par exemple, l'éthionamide ou le prothionamide.

La présente invention concerne également des produits (kits) contenant au moins un des composés précités et au moins un antibiotique actif contre les bactéries et/ou mycobactéries, en particulier un antibiotique activable selon la voie de l'EthA, plus particulièrement un antibiotique choisi parmi la famille des thioamides comme, par exemple, l'éthionamide ou le prothionamide, comme produits de combinaison pour une utilisation simultanée, séparée ou étalée dans le temps en thérapie antituberculeuse, antilépromateuse ou antimycobactérienne générale.

La tuberculose tue 2 millions de personnes chaque année dans le monde. L'épidémie de sida et l'émergence de souches multi résistantes aux antibiotiques contribuent à aggraver l'impact de cette maladie, considérée par l'Organisation Mondiale de la Santé comme responsable d'une épidémie mondiale de plus en plus dangereuse et comme une urgence sanitaire au niveau planétaire.

Un nombre croissant de souches de *Mycobacterium tuberculosis* est aujourd'hui caractérisé par une multi résistance aux antibiotiques de première ligne que sont l'isoniazide (INH) et la rifampicine (RIF). Ces antibiotiques doivent alors être remplacés par des antibiotiques de seconde ligne comme l'éthionamide (ETH) auxquels les souches ne sont pas résistantes mais qui ont le désavantage de présenter un indice thérapeutique faible (l'indice thérapeutique d'un principe actif est le rapport de la dose thérapeutique à la dose toxique).

Une stratégie consistant à augmenter l'activité de l'éthionamide (ETH) en l'associant à un composé particulier a déjà été envisagée. En effet, l'ETH est une prodrogue qui est transformée *in vivo* en une forme thérapeutiquement active par l'enzyme EthA (voir l'article "Activation of the prodrug ethionamide is regulated in mycobacteria", A.R. Baulard et al., Journal of Biological Chemistry, 2000, 275, 28326-28331). Certaines résistances observées à l'ETH viennent du fait que le répresseur transcriptionnel EthR de *M. tuberculosis* contrôle l'expression de l'enzyme EthA et limite la transformation de l'ETH en substance thérapeutiquement active.

Un but de la présente invention est de proposer de nouveaux composés susceptibles de potentialiser notamment, l'activité d'antibiotiques actifs contre la tuberculose, en particulier un antibiotique choisi dans la famille des thioamides, comme l'éthionamide ou le prothionamide, par exemple.

Un autre but de la présente invention est de proposer des composés tels que précités qui permettent notamment en combinaison avec un antibiotique actif contre la tuberculose, choisi dans la famille des thioamides, en particulier, l'éthionamide et/ou le prothionamide, d'obtenir à dose d'antibiotique égale une meilleure efficacité ou qui permettent de réduire la dose d'antibiotique précitée pour obtenir une efficacité donnée.

Un autre but de la présente invention est de proposer des composés tels que précités qui soient simples et peu coûteux à produire.

Un autre but de la présente invention est de proposer des composés tels que précités qui soient solubles de manière satisfaisante dans un fluide biologique.

Un autre but de la présente invention est de proposer des composés tels que précités qui sont susceptibles d'être actifs en particulier par voie orale et/ou qui engendrent moins d'effets secondaires.

Pour atteindre au moins un des objectifs précités, la présente invention propose donc des composés de formule générale (I) : 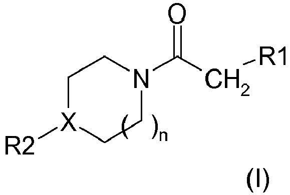 dans laquelle :
n = 0 ou 1 ;
R1 représente un groupement -CH₂CF₃ ou -CH₂CH₂CF₃;
X est choisi parmi N et CH ;
R2 est choisi parmi les groupements suivants:
   i. phényle;
   ii. benzyle;
   iii. phényle substitué par au moins
      a. une chaîne alkyle en C1-C4 linéaire ou ramifiée;
      b. une chaîne alkyle en C1-C4 linéaire ou ramifiée et substituée par au moins un atome de fluor (F);
      c. un groupement choisi parmi Cl, F, CF₃, OCH₃ et OH.
   iv. benzyle substitué par au moins
      a.une chaîne alkyle en C1-C4 linéaire ou ramifiée;
      b. une chaîne alkyle en C1-C4 linéaire ou ramifiée et substituée par au moins un atome de fluor (F); et
   v. les hétérocycles à 6 sommets comportant un, deux ou trois atomes d'azote.

Avantageusement n = 1. De tels composés ont une plus grande activité en combinaison avec l'éthionamide sur les mycobactéries, en particulier sur *M. tuberculosis.*

Avantageusement, R1 est un groupement -CH₂CF₃. De tels composés présentent une bonne activité potentialisatrice de l'éthionamide, en particulier sur *M. tuberculosis.*

Avantageusement, X = CH. De tels composés se sont révélés être plus efficaces en combinaison avec l'éthionamide, en particulier sur la bactérie *M. tuberculosis.*

Selon un premier mode de réalisation, R2 est un groupement phényle.

Selon un second mode de réalisation, R2 est un groupement benzyle.

Selon un troisième mode de réalisation, R2 est un groupement phényle substitué par au moins un atome F.

Selon un quatrième mode de réalisation, R2 est un groupement phényle substitué en position méta par rapport à liaison à X, par Cl, F, CF₃ ou CH₃.

Avantageusement, R2 est un groupement phényle substitué en position para par rapport à la liaison à X, par un atome de fluor F.

Selon un autre mode de réalisation, R2 est un groupement choisi parmi les groupements suivants :

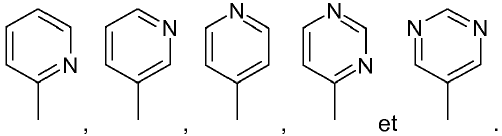

Le composé de l'invention peut être choisi parmi les composés suivants :

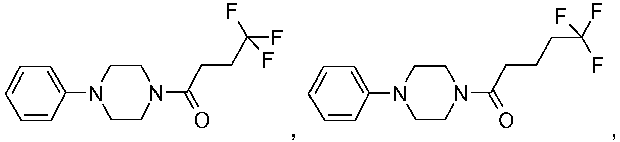

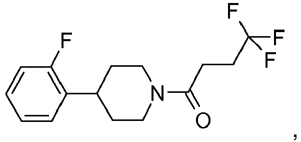

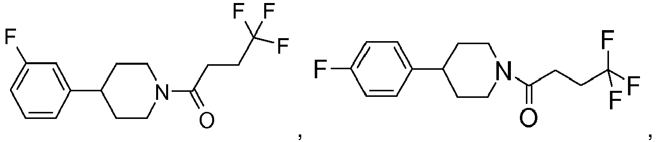

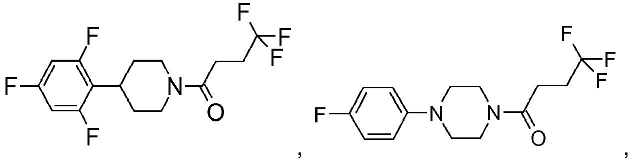

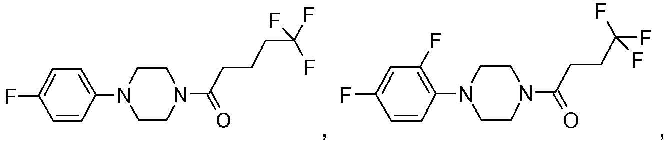

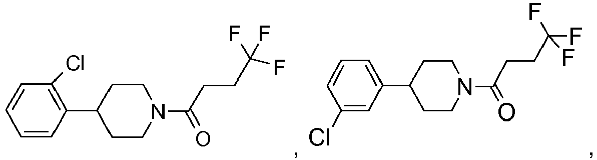

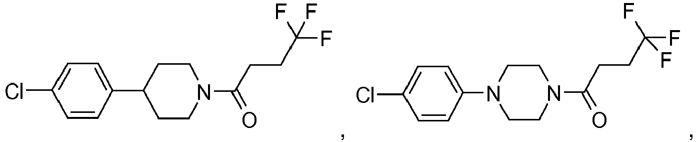

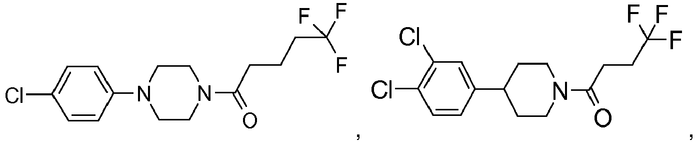

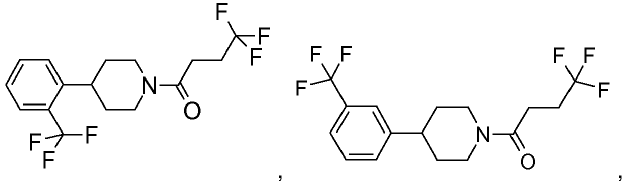

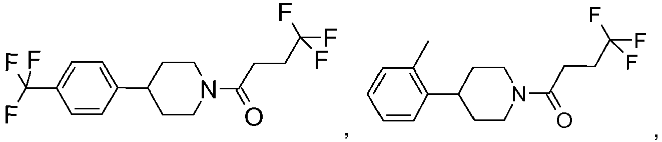

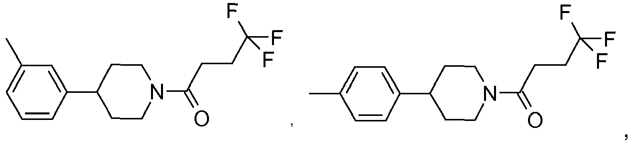

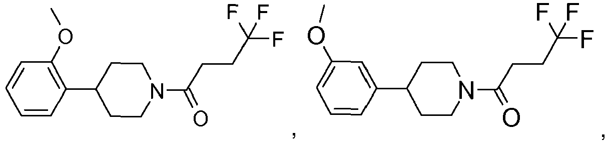

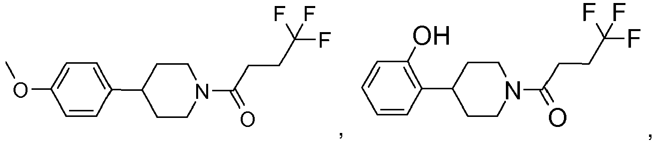

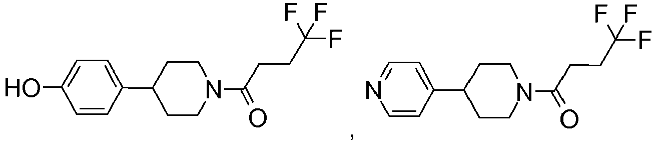

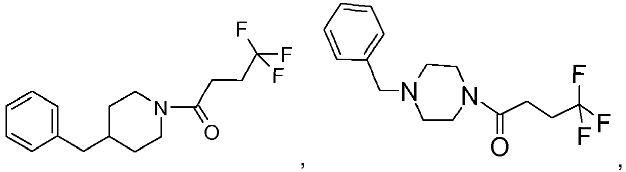

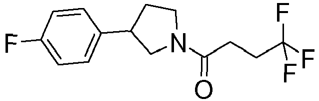

La présente invention concerne également le composé précité pour son utilisation en tant que médicament, en particulier, pour son utilisation dans le traitement des infections bactériennes et mycobactériennes, notamment dans le traitement de la tuberculose, de la lèpre ou de mycobactérioses atypiques.

La présente invention concerne également une composition pharmaceutique comprenant, en tant que principe actif, au moins un composé de formule générale (I) telle que précitée et un excipient pharmaceuticalement acceptable.

Au sein des compositions pharmaceutiques conformes à l'invention, le ou les composés utilisés en tant qu'ingrédient(s) actif(s) peuvent être utilisés en une quantité permettant d'administrer des doses unitaires comprises entre 0,3 mg et 1 g environ. Au sein des compositions pharmaceutiques conformes à l'invention et lorsqu'il(s)est(sont) présent(s), le ou les antibiotiques actifs contre les mycobactéries sont avantageusement utilisés en une quantité permettant d'administrer des doses unitaires égales ou inférieures aux doses habituellement recommandées par l'OMS (WHO, Treatment of tuberculosis: Guidelines for National Programmes. 2003; WHO/CDS/TB2003.313.), les organisations sanitaires nationales ou non gouvernementales, ou les laboratoires pharmaceutiques compétents.

L'Homme du Métier est à même de choisir un ou plusieurs excipients pharmaceutiquement acceptables en fonction de la voie d'administration de la composition pharmaceutique. Bien entendu, l'Homme du Métier veillera à cette occasion à ce que le ou les excipients utilisés soient compatibles avec les propriétés intrinsèques attachées à la composition conforme à la présente invention. En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente invention, le médicament ou la composition pharmaceutique peut être administré(e) par n'importe quelle voie appropriée, par exemple par la voie orale, anale, locale (topique, par exemple), systémique, intraveineuse, intramusculaire ou mucosale, ou bien en utilisant un patch, ou encore sous forme encapsulée dans, ou immobilisée sur, des liposomes, des microparticules, des microcapsules, associée à des nanoparticules et analogues. On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc....Les techniques de formulation et d'administration des médicaments et compositions pharmaceutiques sont bien connues dans la technique ici considérée, l'Homme du Métier pouvant notamment se référer à l'ouvrage Remington's Pharmaceutical Sciences, dernière édition.

La présente invention a également pour objet l'utilisation d'au moins un composé selon l'invention pour la fabrication d'un médicament destiné à la prévention et/ou au traitement des infections bactériennes, de préférence mycobactériennes, et tout particulièrement de la tuberculose, de la lèpre ou de mycobactérioses atypiques.

Avantageusement, la composition pharmaceutique comprend en outre, en tant que principe actif, au moins un antibiotique actif contre les bactéries et/ou mycobactéries, en particulier un antibiotique activable selon la voie de l'EthA, plus particulièrement un antibiotique choisi notamment parmi la famille des thioamides, en particulier parmi l'éthionamide et le prothionamide.

La présente invention n'est néanmoins pas limitée à ces antibiotiques.

Les composés de l'invention s'avèrent être des composés potentialisateurs d'antibiotiques activables par la voie EthA ; néanmoins les composés de l'invention peuvent également être utilisés en tant qu'agent potentialisateurs de l'activité antibiotique d'antibiotiques bioactivables par une ou d'autres voie(s) d'activation que celle précitée.

La présente invention concerne également un kit ou produit contenant au moins un composé de formule (I) et au moins un antibiotique actif contre les bactéries et/ou mycobactéries en particulier, en particulier un antibiotique activable selon la voie de l'EthA, plus particulièrement un antibiotique choisi parmi la famille des thioamides, en particulier choisi parmi l'éthionamide et le prothionamide comme produits de combinaison pour une utilisation simultanée, séparée ou étalée dans le temps en thérapie antituberculeuse, anti-lépromateuse ou anti-mycobactérienne générale.

### DEFINITIONS

Dans toute la présente demande, s'il n'est pas précisé que le groupement quel qu'il soit est substitué, ce dernier n'est pas substitué.

On définit au sens de la présente invention un groupement phényle substitué comme étant un groupement phényle mono-, di- ou tri-substitué. La position du ou des substituants, lorsqu'elle n'est pas précisée n'est pas limitée selon l'invention. Lorsque le ou les substituants sont indiqués, le groupement phényle peut également comprendre un ou plusieurs autres substituants différents de celui cités.

De préférence, les groupements phényles substitués par Cl, CF₃, et CH₃ sont monosubstitués et préférentiellement le substituant (Cl, CF₃ ou CH₃) est en position méta par rapport au carbone du cycle benzénique lié à X. De préférence X est CH.

Dans le cas d'un groupement phényle substitué par un atome de fluor, tous les groupements phényles mono-, di- ou tri-substitués par des atomes de fluor sont inclus dans la présente invention. Avantageusement, les groupements phényles substitués par un ou plusieurs atomes de fluor ne sont pas substitués par un autre groupement ou par un autre atome autre que F. Ainsi, les groupements phényle substitués par au moins un atome de fluor incluent, au sens de la présente invention, les groupement phényles monosubstitués par un atome de fluor, situé en position ortho, méta ou para du carbone du cycle benzénique lié à X, les groupements phényles substitués par deux atomes de fluor, en particulier les groupements phényles substitués par deux atomes de fluor disposés en position ortho et para de la liaison du cycle benzénique avec X, les groupements phényles dont trois atomes de carbones sont substitués chacun par un atome de fluor, en particulier un groupement phényle trisubstitué par trois atomes de fluor dont deux atomes de fluor sont en ortho de la liaison du cycle benzénique avec X et un atome de fluor est en para par rapport à cette liaison.

On définit ici les mycobactérioses atypiques comme étant les mycobactérioses causées par au moins une mycobactérie autre que *M. Tuberculinum* et en particulier les mycobactérioses impliquant *M. Kansasii.*

Au sens de la présente invention, le terme « traitement » désigne le traitement curatif et/ou le traitement prophylactique des infections précitées. Le terme « traitement » englobe toute amélioration de l'état du patient, en particulier toute diminution de la quantité de bactéries présentes dans au moins un site d'infection du patient.

On définit au sens de la présente invention, un antibiotique actif contre les bactéries et/ou mycobactéries, tout agent susceptible de limiter ou de réduire au moins *in vitro* la prolifération d'une bactérie et/ou d'une mycobactérie, en particulier *M. tuberculosis.* Un agent susceptible de détruire, au moins *in vitro* une mycobactérie, notamment M. *tuberculosis* est également un antibiotique actif contre les mycobactéries, au sens de la présente invention. Parmi les antibiotiques actifs contre les mycobactéries, on peut citer l'éthionamide, le prothionamide, l'isoxyl, le thiacétazone et les mélanges d'au moins deux de ces antibiotiques.

On définit au sens de la présente invention, un antibiotique activable par la voie de l'EthA, toute substance qui, au moins *in vitro* réagit avec l'enzyme EthA pour produire une substance ayant des propriétés antibiotiques. L'Homme du métier est à même de déterminer si un antibiotique est activable par la voie de l'EthA, par exemple, en appliquant la méthode décrite dans la publication suivante : "Activation of the prodrug ethionamide is regulated in mycobacteria" 2000 Journal of Biological Chemistry

L'antibiotique au sens la présente invention peut aussi être un antibiotique activable par une autre voie de bioactivation que celle précitée.

### PARTIE EXPERIMENTALE

### SYNTHESES

Les spectres RMN ¹H et ¹³C ont été réalisés à température ambiante sur un appareil Bruker™ DPX 300 à 300 MHz. Les déplacements chimiques sont indiqués en partie par million (ppm). Les assignements ont été réalisés en utilisant les expériences à une dimension (1D) ¹H et ¹³C ou à deux dimensions (2D) HSQC et COSY. Les spectres de masse ont été réalisés sur une LCMS Waters Alliance Micromass ZQ 2000. Les réactifs et solvants commerciaux ont été utilisés sans purification ultérieure.

### Schéma général de synthèse des dérivés pipéridines et pyrrolidines :

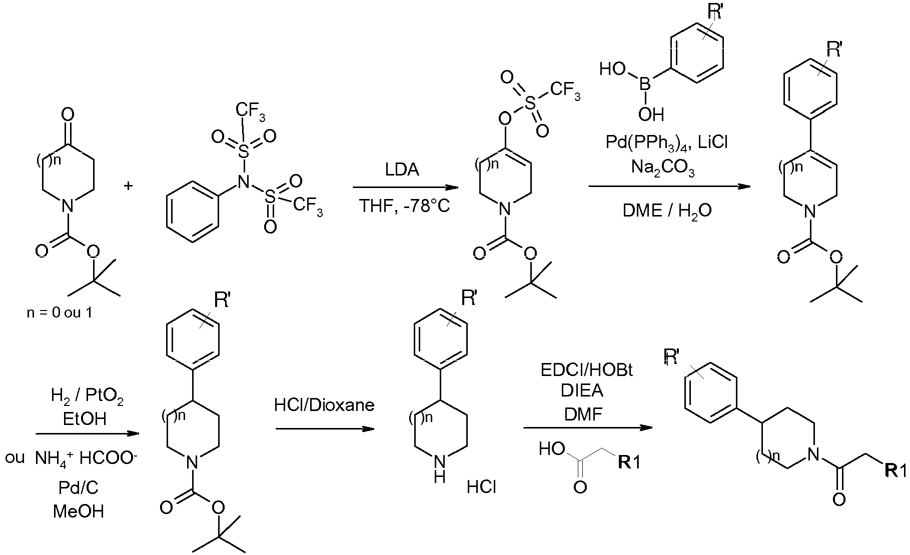

### Protocole :

La LDA (solution à 2M dans THF/heptane/ethylbenzene, 3.3 mmole, 1.1 eq) est introduite avec 5 mL de THF anhydre dans un ballon préalablement séché à l'étuve et mis sous argon. La solution est refroidie à -78°. La N-Boc-4-piperidone (ou la N-Boc-3-pyrrolidinone) (3 mmol, 1 eq) dissoute dans 5 mL de THF est ajoutée goutte à goutte puis le milieu réactionnel est agité 20 min à -78°C. Le N-phenyltrifluoromethanesulfonimide (3.3 mmol, 1.1 eq) dissous dans 5 mL de THF est ajouté. La solution est agitée 2h à 0°C puis évaporée. Le résidu est dissous dans un mélange cyclohexane/AcOEt 9:1 puis filtré sur alumine. Le produit (triflate) est utilisé dans l'étape suivante sans purification.

Dans un ballon contenant le triflate (1 eq) et mis sous argon on introduit l'acide boronique (1.1 eq), LiCl (3 eq), la solution 2N de Na₂CO₃ (1.4 eq), le DME (0.34 M) et le tetrakis(triphenylphosphine)palladium (0.05 eq). La solution est chauffée entre 1h et 16h à reflux puis évaporée. Le résidu est repris dans l'AcOEt puis lavé une fois à l'eau et une fois avec une solution saturée de NaCl. La phase organique est séchée puis évaporée. Le résidu est repris dans l'AcOEt puis filtré sur verre fritté. Le solvant est évaporé puis le produit est purifié par chromatographie sur gel de silice (cyclohexane/AcOEt).

Le dérivé insaturé (1 eq) est dissous dans l'éthanol (0.1M) avec PtO₂ (0.1 eq) ou Pd/C (0.1 eq). Le mélange réactionnel est mis sous hydrogène puis agité à température ambiante jusqu'à disparition du produit de départ. La solution est filtrée sur célite puis évaporée.

Ou le dérivé insaturé (1 eq) est dissous dans le méthanol (0.1M) avec du formate d'ammonium (5 eq) et du Pd/C (10% en masse). Le mélange réactionnel est chauffé à reflux jusqu'à disparition du produit de départ. La solution est filtrée sur célite puis évaporée.

L'amine protégée (1 eq) est introduite dans un ballon avec du dioxane (1 M) puis une solution d'HCl 4N dans le dioxane (5 eq) est ajoutée. La solution est agitée 1h à température ambiante puis évaporée. Le résidu est repris dans l'éther de pétrole puis filtré sur verre fritté.

L'acide (1.3 eq) est activé avec EDCl (1.3 eq) et HOBt (0.4 eq) dans le DMF (0.25 M) en présence de DIEA (4 eq) puis l'amine (1 eq) est ajoutée. La solution est agitée 3h à température ambiante puis évaporée. Le résidu est dissous dans l'AcOEt puis lavé deux fois avec NaHCO₃ saturée, deux fois avec HCl 1N et une fois avec NaCl saturée. La phase organique est séchée sur MgSO₄ puis évaporée. Le résidu est purifié par HPLC préparative.

### Schéma général de synthèse des pipérazines :

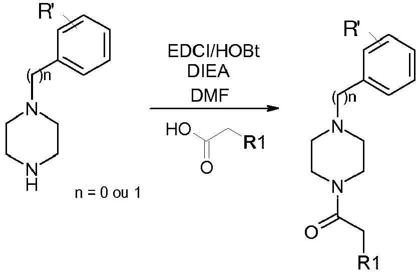

### Protocole :

L'acide (1.3 eq) est activé avec EDCl (1.3 eq) et HOBt (0.4 eq) dans le DMF (0.25 M) en présence de DIEA (4 eq) puis la pipérazine commercialement disponible (1 eq) est ajoutée. La solution est agitée 3h à température ambiante puis évaporée. Le résidu est dissous dans l'AcOEt puis lavé deux fois avec NaHCO₃ saturée, deux fois avec HCl 1N et une fois avec NaCl saturée. La phase organique est séchée sur MgSO₄ puis évaporée. Le résidu est purifié par HPLC préparative.

### BDM_44647

La 4-phenylpiperidine est commercialement disponible. Seul le couplage a été réalisé.

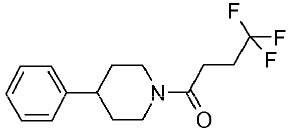

¹H NMR (CD₂Cl₂) δ 7.36-7.31 (m, 2H), 7.25-7.21 (m, 3H), 4.78-4.71 (m, 1H), 3.99-3.93 (m, 1H), 3.22-3.12 (m. 1H), 2.84-2.48 (m, 6H), 1.96-1.86 (m, 2H), 1.72-1.56 (m, 2H). MS [M + H]⁺ *m*/*z* 286.

### BDM_44648

La 4-phenylpiperidine est commercialement disponible. Seul le couplage a été réalisé.

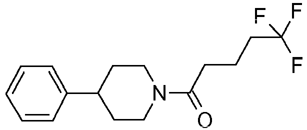

¹H NMR (CD₂Cl₂) δ 7.36-7.31 (m, 2H), 7.25-7.20 (m, 3H), 4.78-4.72 (m, 1H), 3.99-3.92 (m, 1H), 3.19-3.09 (m, 1H), 2.81-2.60 (m, 2H), 2.45 (t, J = 7.0 Hz, 2H), 2.29-2.16 (m, 2H), 1.97-1.87 (m, 4H), 1.70-1.54 (m, 2H). MS [M + H]⁺ *m*/*z* 300.

### BDM_44808

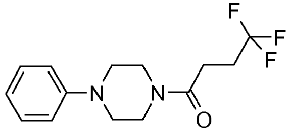

¹H NMR (CDCl₃) δ 7.34-7.28 (m, 2H), 6.97-6.94 (m, 3H), 3.83-3.80 (m, 2H), 3.67-3.64 (m, 2H), 3.24-3.17 (m, 4H), 2.68-2.49 (m, 4H). MS [M + H]⁺ *m*/*z* 287.

### BDM_44809

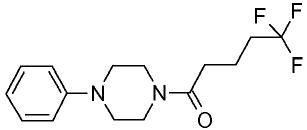

¹H NMR (CDCl₃) δ 7.34-7.28 (m, 2H), 6.97-6.94 (m, 3H), 3.82-3.79 (m, 2H), 3.65-3.62 (m, 2H), 3.22-3.16 (m, 4H), 2.48 (t, J = 7.2 Hz, 2H), 2.31-2.15 (m, 2H), 2.03-1.92 (m, 2H). MS [M + H]⁺ *m*/*z* 301.

### BDM_70666

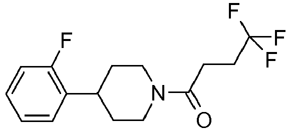

¹H NMR (CD₂Cl₂) δ 7.27-7.20 (m, 2H), 7.16-7.03 (m, 2H), 4.79-4.73 (m, 1H), 3.98-3.93 (m, 1H), 3.24-3.08 (m, 2H), 2.74-2.48 (m, 5H), 1.95-1.86 (m, 2H), 1.74-1.63 (m, 2H). MS [M + H]⁺ *m*/*z* 304.

### BDM_70531

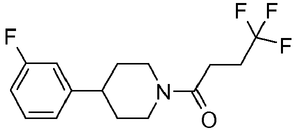

¹H NMR (CD₂Cl₂) δ 7.27-7.20 (m, 2H), 7.16-7.02 (m, 2H), 4.79-4.74 (m, 1H), 3.99-3.94 (m, 1H), 3.24-3.09 (m, 2H), 2.75-2.49 (m, 5H), 1.95-1.86 (m, 2H), 1.75-1.59 (m, 2H). MS [M + H]⁺ *m*/*z* 304.

### BDM_44751

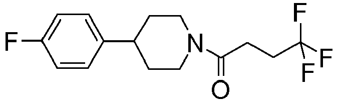

¹H NMR (CD₂Cl₂) δ 7.33-7.19 (m, 2H), 7.06-7.00 (m, 2H), 4.77-4.72 (m, 1H), 3.98-3.92 (m, 1H), 3.21-3.11 (m, 1H), 2.83-2.49 (m, 6H), 1.94-1.86 (m, 2H), 1.68-1.46 (m, 2H).

¹³C NMR (CD₂Cl₂) δ 167.64, 161.45 (d, J = 244 Hz), 141.20, 127.42 (q, J = 274 Hz), 128.16 (d, J = 8 Hz), 115.11 (d, J = 21 Hz), 45.83, 42.40, 41.91, 33.81, 32.96, 29.53 (q, J = 29 Hz), 25.79. MS [M + H]⁺ *m*/*z* 304.

### BDM_71148

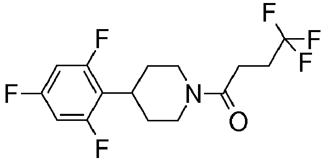

¹H NMR (CD₂Cl₂) δ 6.69 (t, J = 8.7 Hz, 2H), 4.78-4.72 (m, 1H), 3.98-3.92 (m, 1H), 3.27-3.10 (m, 2H), 2.68-2.48 (m, 5H), 2.07-1.90 (m, 2H), 1.82-1.74 (m, 2H). MS [M + H]⁺ *m*/*z* 340.

### BDM_44819

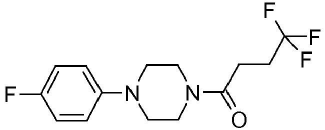

¹H NMR (CD₂Cl₂) δ 7.04-6.98 (m, 2H), 6.95-6.90 (m, 2H), 3.79-3.75 (m, 2H), 3.64-3.61 (m, 2H), 3.14-3.07 (m, 4H), 2.61-2.46 (m, 4H). MS [M + H]⁺ *m*/*z* 305.

### BDM_44820

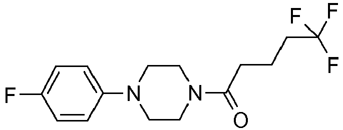

¹H NMR (CD₂Cl₂) δ 7.04-6.98 (m, 2H), 6.94-6.89 (m, 2H), 3.77-3.74 (m, 2H), 3.62-3.59 (m, 2H), 3.12-3.06 (m, 4H), 2.45 (t, J = 7.2 Hz, 2H), 2.25-2.15 (m, 2H), 1.97-1.87 (m, 2H). MS [M + H]⁺ *m*/*z* 319.

### BDM_70669

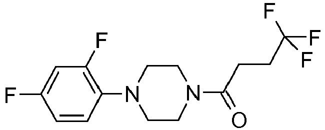

¹H NMR (CD₂Cl₂) δ 6.99-6.82 (m, 3H), 3.79-3.76 (m, 2H), 3.64-3.61 (m, 2H), 3.05-2.99 (m, 4H), 2.67-2.48 (m, 4H). MS [M + H]⁺ *m*/*z* 323.

### BDM_70534

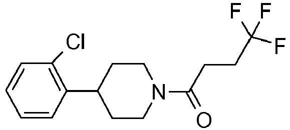

¹H NMR (CD₂Cl₂) δ 7.41-7.39 (m, 1H), 7.32-7.17 (m, 3H), 4.80-4.75 (m, 1H), 3.99-3.94 (m, 1H), 3.35-3.17 (m, 2H), 2.76-2.49 (m, 5H), 1.99-1.89 (m, 2H), 1.66-1.53 (m, 2H). MS [M + H]⁺ *m*/*z* 320.

### BDM_70668

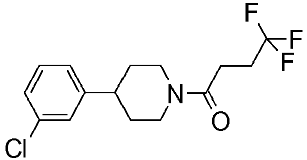

¹H NMR (CD₂Cl₂) δ 7.32-7.21 (m, 3H), 7.15-7.13 (m, 1H), 4.78-4.72 (m, 1H), 3.98-3.93 (m, 1H), 3.21-3.11 (m, 1H), 2.83-2.48 (m, 6H), 1.95-1.87 (m, 2H), 1.69-1.52 (m, 2H). MS [M + H]⁺ *m*/*z* 320.

### BDM_70535

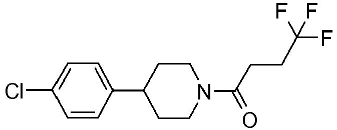

¹H NMR (CD₂Cl₂) δ 7.33-7.30 (m, 2H), 7.20-7.17 (m, 2H), 4.78-4.71 (m, 1H), 3.99-3.92 (m, 1H), 3.21-3.11 (m, 1H), 2.82-2.48 (m, 6H), 1.94-1.86 (m, 2H), 1.67-1.51 (m, 2H). MS [M + H]⁺ *m*/*z* 320.

### BDM_44811

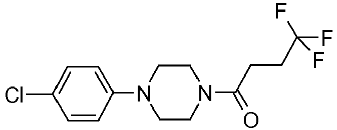

¹H NMR (CD₂Cl₂) δ 7.28-7.23 (m, 2H), 6.91-6.86 (m, 2H), 3.78-3.75 (m, 2H), 3.64-3.61 (m, 2H), 3.20-3.13 (m, 4H), 2.67-2.46 (m, 4H). MS [M + H]⁺ *m*/*z* 321.

### BDM_44812

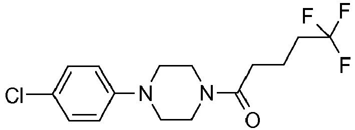

¹H NMR (CD₂Cl₂) δ 7.27-7.22 (m, 2H), 6.91-6.86 (m, 2H), 3.77-3.74 (m, 2H), 3.62-3.59 (m, 2H), 3.18-3.12 (m, 4H), 2.45 (t, J = 7.2 Hz, 2H), 2.31-2.15 (m, 2H), 1.97-1.87 (m, 2H). MS [M + H]⁺ *m*/*z* 335.

### BDM_70716

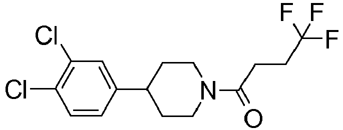

¹H NMR (CDCl₃) δ 7.39 (d, J = 8.3 Hz, 1H), 7.29 (d, J = 2.0 Hz, 1H), 7.04 (dd, J = 8.3 Hz, J = 2.0 Hz, 1H), 4.82-4.77 (m, 1H), 4.00-3.94 (m, 1H), 3.21-3.12 (m, 1H), 2.79-2.48 (m, 6H), 1.96-1.88 (m, 2H), 1.67-1.51 (m, 2H).

¹³C NMR (CDCl₃) δ 167.99, 145.10, 132.60, 130.58, 128.84, 127.11 (q, J = 275 Hz), 126.11, 45.74, 42.40, 41.90, 33.48, 32.53, 29.69 (q, J = 29 Hz), 25.95. MS [M + H]⁺ *m*/*z* 354.

### BDM_70536

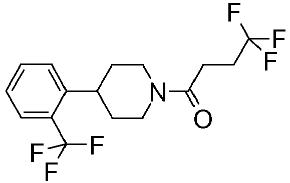

¹H NMR (CD₂Cl₂) δ 7.68 (d, J = 4.5 Hz, 1H), 7.58 (t, J = 4.5 Hz, 1H), 7.46 (d, J = 4.5 Hz, 1H), 7.37 (t, J = 4.5 Hz, 1H), 4.81-4.77 (m, 1H), 4.00-3.97 (m, 1H), 3.23-3.17 (m, 2H), 2.73-2.52 (m, 5H), 1.92-1.85 (m, 2H), 1.75-1.68 (m, 2H). MS [M + H]⁺ *m*/*z* 354.

### BDM_70546

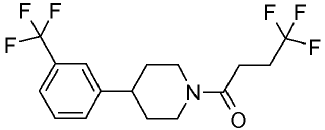

¹H NMR (CD₂Cl₂) δ 7.51-7.46 (m, 4H), 4.80-4.75 (m, 1H), 4.01-3.95 (m, 1H), 3.23-3.14 (m, 1H), 2.93-2.82 (m, 1H), 2.74-2.50 (m, 5H), 1.99-1.90 (m, 2H), 1.74-1.57 (m, 2H).

¹³C NMR (CD₂Cl₂) δ 167.72, 146.30, 130.56 (q, J = 32 Hz), 130.37, 129.09, 127.44 (q, J = 275 Hz), 124.35 (q, J = 275 Hz), 123.51 (q, J = 4 Hz), 123.25 (q, J = 4 Hz), 45.73, 42.48, 42.29, 33.46, 32.63, 29.52 (q, J = 28 Hz), 25.83. MS [M + H]⁺ *m*/*z* 354.

### BDM_70667

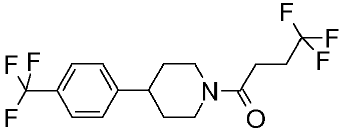

¹H NMR (CD₂Cl₂) δ 7.61 (d, J = 8.4 Hz, 2H), 7.37 (d, J = 8.4 Hz, 2H), 4.80-4.74 (m, 1H), 4.01-3.95 (m, 1H), 3.23-3.13 (m, 1H), 2.92-2.82 (m, 1H), 2.73-2.48 (m, 5H), 1.98-1.89 (m, 2H), 1.73-1.61 (m, 2H). MS [M + H]⁺ *m*/*z* 354.

### BDM_70665

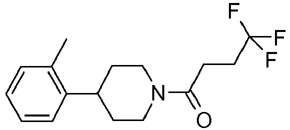

¹H NMR (CD₂Cl₂) δ 7.19-7.09 (m, 4H), 4.80-4.74 (m, 1H), 4.00-3.94 (m, 1H), 3.23-3.14 (m, 1H), 3.06-2.95 (m, 1H), 2.74-2.47 (m, 5H), 2.38 (s, 3H), 1.88-1.80 (m, 2H), 1.71-1.54 (m, 2H). MS [M + H]⁺ *m*/*z* 300.

### BDM_70664

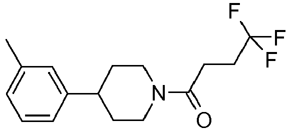

¹H NMR (CD₂Cl₂) δ 7.23-7.20 (m, 1H), 7.06-7.01 (m, 3H), 4.77-4.71 (m, 1H), 3.98-3.92 (m, 1H), 3.20-3.11 (m, 1H), 2.79-2.46 (m, 6H), 2.33 (s, 3H), 1.94-1.85 (m, 2H), 1.71-1.53 (m, 2H). MS [M + H]⁺ *m*/*z* 300.

### BDM_70663

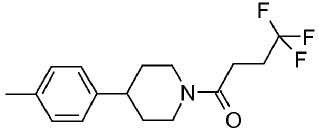

¹H NMR (CD₂Cl₂) δ 7.16-7.10 (m, 4H), 4.77-4.70 (m, 1H), 3.97-3.91 (m, 1H), 3.20-3.10 (m, 1H), 3.06-2.95 (m, 1H), 2.77-2.47 (m, 5H), 2.33 (s, 3H), 1.93-1.85 (m, 2H), 1.69-1.51 (m, 2H). MS [M + H]⁺ *m*/*z* 300.

### BDM_70540

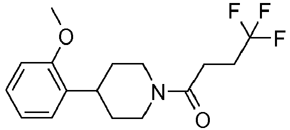

¹H NMR (CD₂Cl₂) δ 7.25-7.15 (m, 2H), 6.98-6.91 (m, 2H), 4.78-4.72 (m, 1H), 3.97-3.92 (m, 1H), 3.86 (s, 3H), 3.28-3.14 (m, 2H), 2.75-2.47 (m, 5H), 1.94-1.84 (m, 2H), 1.69-1.54 (m, 2H).

¹³C NMR (CD₂Cl₂) δ 167.59, 156.89, 133.37, 127.49 (q, J = 275 Hz), 127.18, 126.35, 120.56, 110.44, 55.23, 46.16, 42.73, 35.54, 32.31, 31.48, 29.59 (q, J = 29 Hz), 25.81. MS [M + H]⁺ *m*/*z* 316.

### BDM_70538

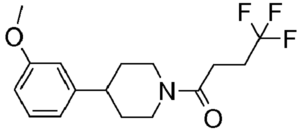

¹H NMR (CD₂Cl₂) δ 7.27-7.22 (m, 1H), 6.83-6.76 (m, 3H), 4.78-4.71 (m, 1H), 3.99-3.92 (m, 1H), 3.80 (s, 3H), 3.20-3.11 (m, 1H), 2.81-2.47 (m, 6H), 1.95-1.87 (m, 2H), 1.71-1.54 (m, 2H). MS [M + H]⁺ *m*/*z* 316

### BDM_70537

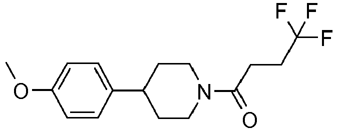

¹H NMR (CD₂Cl₂) δ 7.17-7.14 (m, 2H), 6.89-6.86 (m, 2H), 4.76-4.71 (m, 1H), 3.97-3.92 (m, 1H), 3.79 (s, 3H), 3.20-3.11 (m, 1H), 2.74-2.50 (m, 6H), 1.93-1.86 (m, 2H), 1.63-1.55 (m, 2H).

¹³C NMR (CD₂Cl₂) δ 168.38, 158.58, 138.06, 127.49 (q, J = 275 Hz), 127.56, 113.81, 55.16, 45.97, 42.53, 41.76, 34.06, 33.12, 29.56 (q, J = 29 Hz), 25.80. MS [M + H]⁺ *m*/*z* 316.

### BDM_70539

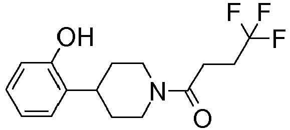

¹H NMR (MeOD) δ 7.08 (dd, J = 7.6 Hz, J = 1.5 Hz, 1H), 7.00 (td, J = 7.6 Hz, J = 1.7 Hz, 1H), 6.80-6.74 (m, 2H), 4.71-4.64 (m, 1H), 4.09-4.02 (m, 1H), 3.27-3.15 (m, 2H), 2.80-2.70 (m, 3H), 2.58-2.47 (m, 2H), 1.96-1.83 (m, 2H), 1.74-1.53 (m, 2H). MS [M + H]⁺ *m*/*z* 302.

### BDM_45572

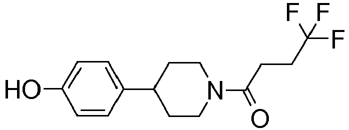

¹H NMR (MeOD) δ 7.04 (d, J = 8.7 Hz, 2H), 6.72 (d, J = 8.7 Hz, 2H), 4.67-4.61 (m, 1H), 4.04-3.98 (m, 1H), 3.21-3.12 (m, 1H), 2.75-2.66 (m, 4H), 2.58-2.46 (m, 2H), 1.89-1.79 (m, 2H), 1.67-1.44 (m, 2H). MS [M + H]⁺ *m*/*z* 302.

### BDM_70542

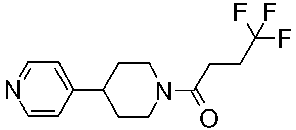

¹H NMR (CD₂Cl₂) δ 8.52 (d, J = 6.1 Hz, 2H), 7.16 (d, J = 6.1 Hz, 2H), 4.80-4.73 (m, 1H), 4.01-3.93 (m, 1H), 3.22-3.13 (m, 1H), 2.84-2.48 (m, 6H), 1.98-1.89 (m, 2H), 1.71-1.54 (m, 2H). MS [M + H]⁺ *m*/*z* 287.

### BDM_70670

La 4-benzylpiperidine est commercialement disponible. Seul le couplage a été réalisé.

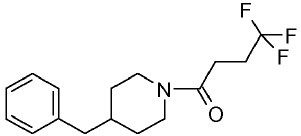

¹H NMR (CD₂Cl₂) δ 7.33-7.28 (m, 2H), 7.24-7.16 (m, 3H), 4.59-4.51 (m, 1H), 3.82-3.77 (m, 1H), 3.02-2.92 (m, 1H), 2.59-2.47 (m, 7H), 1.85-1.67 (m, 3H), 1.24-1.07 (m, 2H). MS [M + H]⁺ *m*/*z* 300.

### BDM_70719

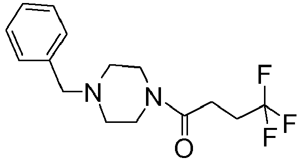

¹H NMR (CD₂Cl₂) δ 7.35-7.26 (m, 5H), 3.61 (t, J = 5.1 Hz, 2H), 3.54 (s, 2H), 3.45 (t, J = 5.1 Hz, 2H), 2.61-2.41 (m, 8H). MS [M + H]⁺ *m*/*z* 301.

### BDM_70717

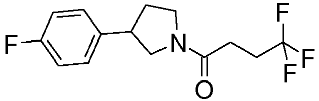

¹H NMR (CDCl₃) δ 7.24-7.18 (m, 2H), 7.07-7.00 (m, 2H), 4-09-3.99 (m, 0.5H), 3.91-3.81 (m, 1H), 3.72-3.64 (m, 0.5H), 3.60-3.31 (m, 3H), 2.61-2.50 (m, 4H), 2.46-2.27 (m, 1H), 2.16-1.95 (m, 1H). MS [M + H]⁺ *m*/*z* 290.

### BDM_44810 (référence)

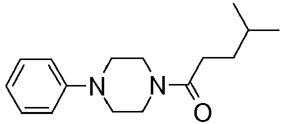

¹H NMR (CDCl₃) δ 7.34-7.28 (m, 2H), 6.97-6.90 (m, 3H), 3.80 (t, J = 5.1 Hz, 2H), 3.66 (t, J = 5.1 Hz, 2H), 3.22-3.15 (m, 4H), 2.42-2.37 (m, 2H), 1.70-1.53 (m, 3H), 0.95 (d, J = 6.3 Hz, 6H). MS [M + H]⁺ *m*/*z* 261.

### BDM_44813 (référence)

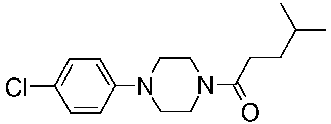

¹H NMR (CDCl₃) δ 7.26-7.22 (m, 2H), 6.91-6.86 (m, 2H), 3.74 (t, J = 5.1 Hz, 2H), 3.63 (t, J = 5.1 Hz, 2H), 3.18-3.11 (m, 4H), 2.39-2.34 (m, 2H), 1.67-1.49 (m, 3H), 0.95 (d, J = 6.3 Hz, 6H). MS [M + H]⁺ *m*/*z* 295.

### BDM_44821 (référence)

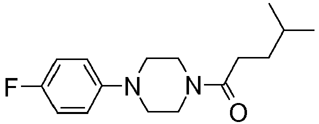

¹H NMR (CDCl₃) δ 7.04-6.98 (m, 2H), 6.94-6.90 (m, 2H), 3.74 (t, J = 5.1 Hz, 2H), 3.63 (t, J = 5.1 Hz, 2H), 3.12-3.05 (m, 4H), 2.39-2.34 (m, 2H), 1.64-1.49 (m, 3H), 0.95 (d, J = 6.6 Hz, 6H). MS [M + H]⁺ *m*/*z* 279.

### BDM_44649 (référence)

La 4-phenylpiperidine est commercialement disponible. Seul le couplage a été réalisé.

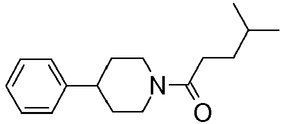

¹H NMR (CD₂Cl₂) δ 7.36-7.31 (m, 2H), 7.25-7.20 (m, 3H), 4.77-4.73 (m, 1H), 4.02-3.98 (m, 1H), 3.18-3.09 (m, 1H), 2.81-2.71 (m, 1H), 2.66-2.57 (m, 1H), 2.39-2.34 (m, 2H), 1.94-1.85 (m, 2H), 1.69-1.51 (m, 5H), 0.95 (d, J = 6.4 Hz, 6H). MS [M + H]⁺ *m*/*z* 260.

### Evaluation de l'activité des composés

### Le test cellulaire de potentialisation de l'éthionamide

Le test utilisé permet de vérifier que ces composés sont capables de potentialiser l'activité bactéricide de l'éthionamide sur *M. tuberculosis* seul. Ce test est un test de « High Content Screening » (HCS) ou criblage à contenu dense. Les tests HCS sont réalisés sur des cultures cellulaires qui permettent d'étudier certains caractères phénotypiques d'un microorganisme (bactérie par exemple) dans un environnement donné. Les changements phénotypiques observés peuvent aller de l'augmentation (ou la diminution) de la production de certaines protéines marquées à la modification de la morphologie du microorganisme étudié. La méthode est décrite dans la publication suivante : "Ethionamide Boosters: Synthesis, Biological Activity, and Structure-Activity Relationships of a Series of 1,2,4-Oxadiazole EthR Inhibitors", M. Flipo et al., Journal of Medicinal Chemistry, 2011, 54(8), 2994-3010.

Ce test a pour but de déterminer la concentration en ligand nécessaire pour potentialiser dix fois l'activité de l'éthionamide (ETH).

Pour mesurer la concentration de ligand nécessaire pour potentialiser dix fois l'activité de l'ETH, on se place à une concentration constante en éthionamide (0.1 µg/mL ce qui correspond au 1/10^{ème} de sa CMI₉₉). En faisant varier la concentration en ligand on peut déterminer la concentration nécessaire pour inhiber 50% de la pousse bactérienne, c'est-à-dire la concentration nécessaire pour potentialiser dix fois l'activité de l'éthionamide. Cette concentration sera notée EC₅₀.

### Mesure de la solubilité

40 µL d'une solution à 10 mM dans le DMSO de l'échantillon est ajoutée à 1.96 mL de MeOH ou de PBS à pH 7.4. Les échantillons sont alors agités pendant 24h à TA, centrifugés pendant 5 min puis filtrés sur des filtres de taille 0.45 µm. 20 µL de chaque solution est alors ajoutée à 180 µL de MeOH puis analysée par LC-MS. La solubilité est déterminée comme le ratio des aires des signaux de masses PBS/MeOH.

### ACTIVITES BIOLOGIQUES MESUREES

Les tableaux I à III suivants regroupent les formules des composés testés ainsi que les valeurs de EC₅₀ et de solubilité mesurées expérimentalement selon les protocoles précités.

**Tableau I**

| **ID_structure** | **R2** | **X** | **n** | **R1** | **EC₅₀ (µM)** | **Solubilité (µg/mL)** |
|---|---|---|---|---|---|---|
| BDM_44647 | 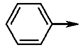 | CH | 1 | CH₂CF₃ | 0.0008 | 50.4 |
| BDM_44648 | 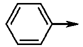 | CH | 1 | (CH₂)₂CF₃ | <0.01 | 42.9 |
| BDM_44751 | 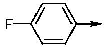 | CH | 1 | CH₂CF₃ | 0.001 | 51.9 |
| BDM_70717 | 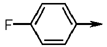 | CH | 0 | CH₂CF₃ | 0.009 | ND |

En référence aux résultats du Tableau I, on constate qu'un groupement CH2CF3 confère une plus grande activité potentialisatrice de l'éthionamide sans nuire à la solubilité du composé.

Les résultats montrent que pour un même radical R1 et un même radical R2, l'activité potentialisatrice des composés de l'invention est améliorée lorsque n =1.

**Tableau II (référence)**

| **ID_structure** | **R2** | **X** | **n** | **R1** | **EC₅₀ (µM)** |
|---|---|---|---|---|---|
| BDM_44810 | 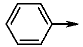 | N | 1 | (CH₂)isopropyle | 0.06 |
| BDM_44813 | 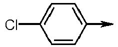 | N | 1 | (CH₂)isopropyle | 0.1 |
| BDM_44821 | 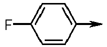 | N | 1 | (CH₂)isopropyle | 0.1 |
| BDM_44649 | 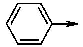 | CH | 1 | (CH₂)isopropyle | 0.06 |

Le Tableau III suivant regroupe les activités exprimés en EC₅₀ pour tous les composés testés.

**Tableau III**

| **ID_structure** | **R2** | **X** | **n** | **R1** | **EC₅₀ (µM)** |
|---|---|---|---|---|---|
| BDM_44647 | 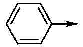 | CH | 1 | CH₂CF₃ | 0.0008 |
| BDM_44648 | 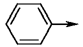 | CH | 1 | (CH₂)₂CF₃ | <0.01 |
| BDM_44649 * | 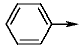 | CH | 1 | (CH₂)isopropyle | 0.06 |
| BDM_44808 | 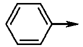 | N | 1 | CH₂CF₃ | 0.01 |
| BDM_44809 | 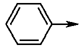 | N | 1 | (CH₂)₂CF₃ | 0.07 |
| BDM_70666 | 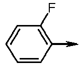 | CH | 1 | CH₂CF₃ | 0.001 |
| BDM_70531 | 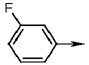 | CH | 1 | CH₂CF₃ | 0.0008 |
| BDM_44751 | 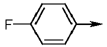 | CH | 1 | CH₂CF₃ | 0.001 |
| BDM_71148 | 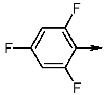 | CH | 1 | CH₂CF₃ | 0.001 |
| BDM_44819 | 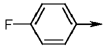 | N | 1 | CH₂CF₃ | 0.027 |
| BDM_44820 | 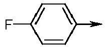 | N | 1 | (CH₂)₂CF₃ | 0.14 |
| BDM_70669 | 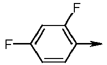 | N | 1 | CH₂CF₃ | 0.021 |
| BDM_70534 | 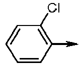 | CH | 1 | CH₂CF₃ | 0.11 |
| BDM_70668 | 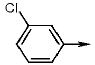 | CH | 1 | CH₂CF₃ | 0.0005 |
| BDM_70535 | 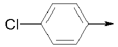 | CH | 1 | CH₂CF₃ | 0.12 |
| BDM_44811 | 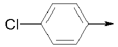 | N | 1 | CH₂CF₃ | 0.010 |
| BDM_44812 | 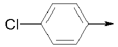 | N | 1 | (CH₂)₂CF₃ | <0.02 |
| BDM_70716 | 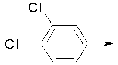 | CH | 1 | CH₂CF₃ | 0.025 |
| BDM_70536 | 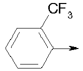 | CH | 1 | CH₂CF₃ | 0.770 |
| BDM_70546 | 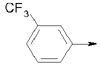 | CH | 1 | CH₂CF₃ | 0.001 |
| BDM_70667 | 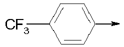 | CH | 1 | CH₂CF₃ | 0.3 |
| BDM_70665 | 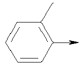 | CH | 1 | CH₂CF₃ | 0.035 |
| BDM_70664 | 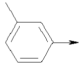 | CH | 1 | CH₂CF₃ | 0.001 |
| BDM_70663 | 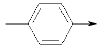 | CH | 1 | CH₂CF₃ | 0.026 |
| BDM_70540 | 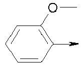 | CH | 1 | CH₂CF₃ | 0.14 |
| BDM_70538 | 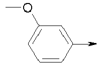 | CH | 1 | CH₂CF₃ | 0.14 |
| BDM_70537 | 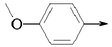 | CH | 1 | CH₂CF₃ | 0.002 |
| BDM_70539 | 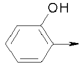 | CH | 1 | CH₂CF₃ | ND |
| BDM_45572 | 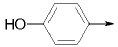 | CH | 1 | CH₂CF₃ | 0.28 |
| BDM_70542 | 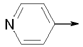 | CH | 1 | CH₂CF₃ | 0.33 |
| BDM_70670 | 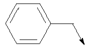 | CH | 1 | CH₂CF₃ | 0.054 |
| BDM_70719 | 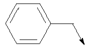 | N | 1 | CH₂CF₃ | 1.1 |
| BDM_44810 * | 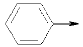 | N | 1 | (CH₂)isopropyle | 0.06 |
| BDM_44813 * | 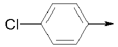 | N | 1 | (CH₂)isopropyle | 0.1 |
| BDM_44821 * | 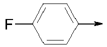 | N | 1 | (CH₂)isopropyle | 0.1 |
| BDM_70717 | 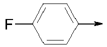 | CH | 0 | CH₂CF₃ | 0.009 |

| | | | | | |
|---|---|---|---|---|---|
| * (référence) | | | | | |

## Revendications

1. Composé de formule (I): 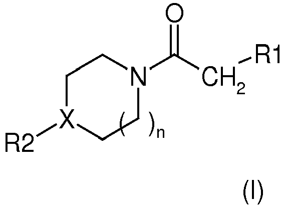 dans laquelle:
n = 0 ou 1 ;
R1 représente un groupement -CH₂CF₃ ou -CH₂CH₂CF₃;
X est choisi parmi N et CH;
R2 est choisi parmi les groupements suivants
i. phényle;
ii. benzyle;
iii. phényle substitués par au moins
a. une chaîne alkyle en C1-C4 linéaire ou ramifiée;
b. une chaîne alkyle en C1-C4 linéaire ou ramifiée et substituée par au moins un atome de fluor (F);
c. un groupement choisi parmi Cl, F, CF₃, OCH₃ et OH;
iv. benzyle substitués par au moins
a. une chaîne alkyle en C1-C4 linéaire ou ramifiée;
b. une chaîne alkyle en C1-C4 linéaire ou ramifiée et substituée par au moins un atome de fluor (F); et
v. les hétérocycles à 6 sommets comportant un, deux ou trois atomes d'azote.

2. Composé selon la revendication 1, **caractérisé en ce que** n = 1.

3. Composé selon la revendication 1 ou 2, **caractérisé en ce que** R1 est un groupement -CH₂CF₃.

4. Composé selon l'une quelconque des revendications précédentes, **caractérisé en ce que** X = CH.

5. Composé selon l'une quelconque des revendications 1 à 4, **caractérisé en ce que** R2 est phényle ou benzyle.

6. Composé selon l'une quelconque des revendications 1 à 4, **caractérisé en ce que** R2 est phényle substitué en position méta par rapport à liaison à X, par un groupement choisi parmi: Cl, F, CF₃ et OCH₃.

7. Composé selon l'une quelconque des revendications 1 à 4, **caractérisé en ce que** R2 est phényle substitué par au moins un atome F.

8. Composé selon l'une quelconque des revendications 1 à 4, **caractérisé en ce que** R2 est phényle substitué en position para par rapport à la liaison à X, par un atome de fluor F.

9. Composé selon l'une quelconque des revendications 1 à 4, **caractérisé en ce que** R2 est un groupement choisi parmi les groupements suivants:

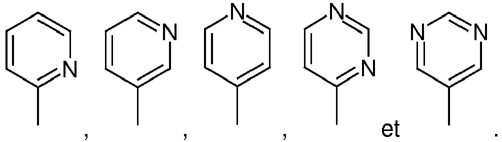

10. Composé selon la revendication 1 **caractérisé en ce qu'**il est choisi parmi les composés suivants: 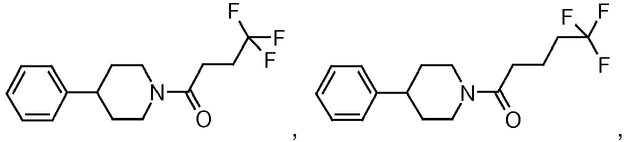 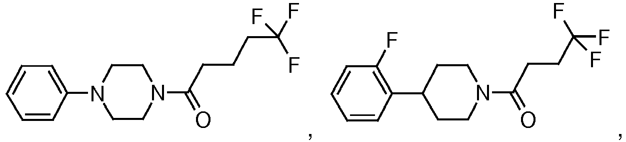 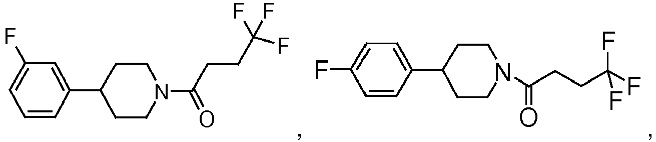 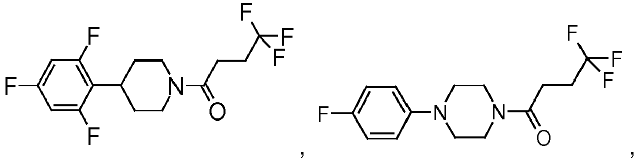 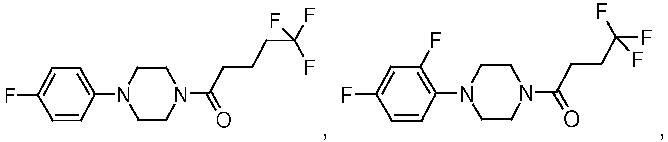 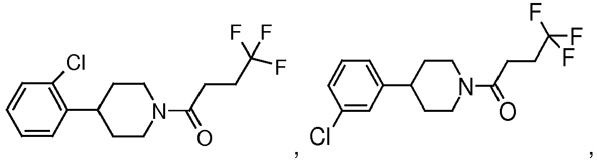 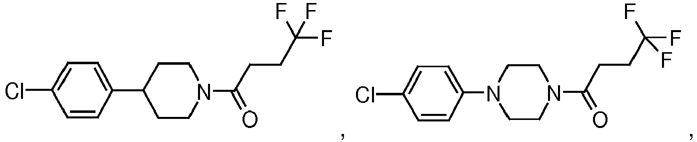 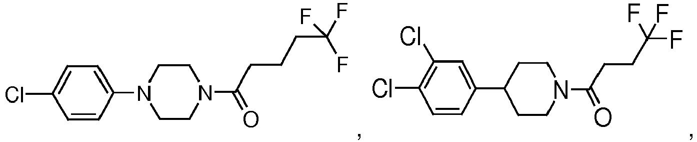 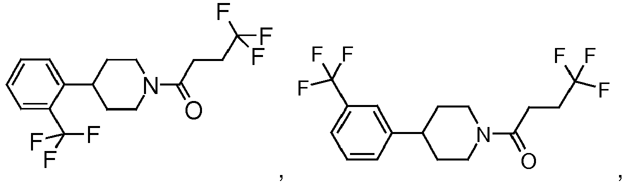 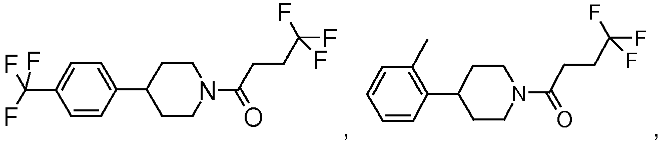 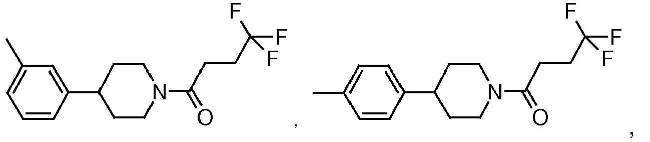 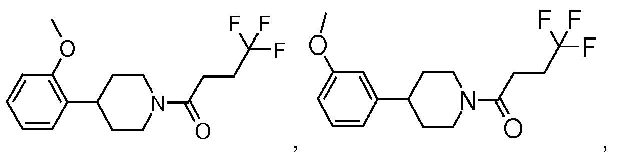 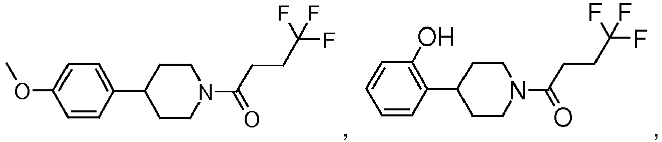 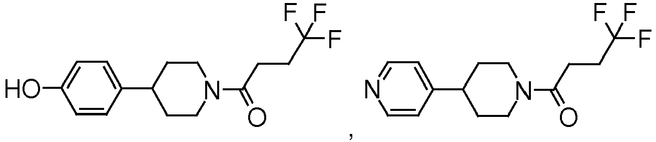 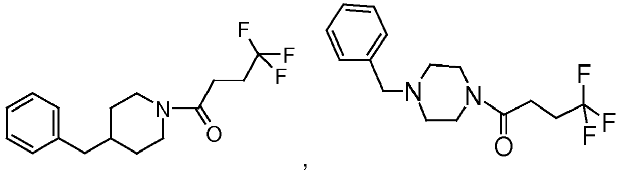 et

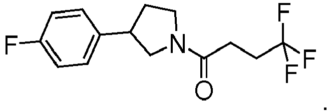

11. Composé selon l'une quelconque des revendications 1 à 10 pour son utilisation dans le traitement de la tuberculose, de la lèpre ou des mycobactérioses atypiques.

12. Composition pharmaceutique comprenant, en tant que principe actif, au moins un composé selon l'une quelconque des revendications 1 à 10 et un excipient pharmaceuticalement acceptable.

13. Composition pharmaceutique comprenant, en tant que principe actif, au moins un composé selon l'une quelconque des revendications 1 à 10, et en outre, en tant que principe actif, au moins un antibiotique activable selon la voie de l'EthA.

14. Composition pharmaceutique selon la revendication 13, comprenant, en tant que principe actif, au moins un composé selon l'une quelconque des revendications 1 à 10, et en outre, en tant que principe actif, au moins un antibiotique choisi parmi la famille des thioamides.

15. Composition pharmaceutique selon la revendication 14, comprenant, en tant que principe actif, au moins un composé selon l'une quelconque des revendications 1 à 10, et en outre, en tant que principe actif, l'éthionamide ou le prothionamide.

16. Produit contenant au moins un composé selon l'une quelconque des revendications 1 à 10 et au moins un antibiotique activable selon la voie de l'EthA, plus particulièrement un antibiotique choisi parmi la famille des thioamides, comme produits de combinaison en thérapie antituberculeuse, anti-lépromateuse ou anti-mycobactérienne générale.

## Patentansprüche

1. Verbindung der Formel (I): 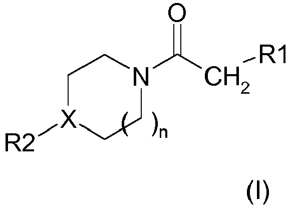 wobei
n = 0 oder 1 gilt;
R1 für eine -CH₂CF₃ oder -CH₂CH₂CF₃-Gruppe steht;
X aus N und CH ausgewählt ist;
R2 aus den folgenden Gruppen ausgewählt ist:
i. Phenyl;
ii. Benzyl;
iii. Phenyl, substituiert mit mindestens
a. einer linearen oder verzweigten C₁₋C₄-Alkylkette;
b. einer mit mindestens einem Fluoratom (F) substituierten linearen oder verzweigten C₁₋C₄₋Alkylkette;
c. einer Gruppe, ausgewählt aus Cl, F, CF₃, OCH₃ und OH;
iv. Benzyl, substituiert mit mindestens
a. einer linearen oder verzweigten C₁₋C₄-Alkylkette;
b. einer mit mindestens einem Fluoratom (F) substituierten linearen oder verzweigten C₁₋C₄-Alkylkette; und
v. den 6-gliedrigen Heterocyclen, die ein, zwei oder drei Stickstoffatome aufweisen.

2. Verbindung nach Anspruch 1, **dadurch gekennzeichnet, dass** n = 1 gilt.

3. Verbindung nach Anspruch 1 oder 2, **dadurch gekennzeichnet**, das R1 eine -CH₂CF₃-Gruppe ist.

4. Verbindung nach einem der vorhergehenden Ansprüche, **dadurch gekennzeichnet, dass** X = CH ist.

5. Verbindung nach einem der Ansprüche 1 bis 4, **dadurch gekennzeichnet, dass** R2 Phenyl oder Benzyl ist.

6. Verbindung nach einem der Ansprüche 1 bis 4, **dadurch gekennzeichnet, dass** R2 Phenyl ist, das metaständig bezüglich der Bindung an X substituiert ist mit einer Gruppe, ausgewählt aus: Cl, F, CF₃ und OCH₃.

7. Verbindung nach einem der Ansprüche 1 bis 4, **dadurch gekennzeichnet, dass** R2 Phenyl ist, das mit mindestens einem F-Atom substituiert ist.

8. Verbindung nach einem der Ansprüche 1 bis 4, **dadurch gekennzeichnet, dass** R2 Phenyl ist, das paraständig bezüglich der Bindung an X mit einem Fluoratom F substituiert ist.

9. Verbindung nach einem der Ansprüche 1 bis 4, **dadurch gekennzeichnet, dass** R2 eine Gruppe ist, die aus den folgenden Gruppen ausgewählt ist:

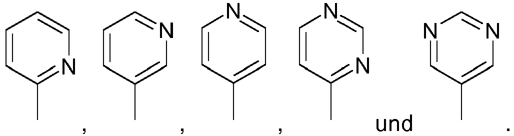

10. Verbindung nach Anspruch 1, **dadurch gekennzeichnet, dass** sie aus den folgenden Verbindungen ausgewählt ist: 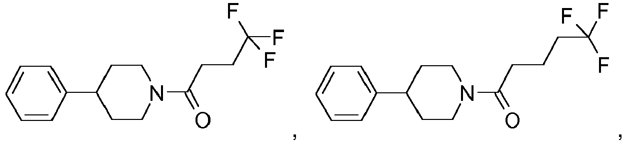 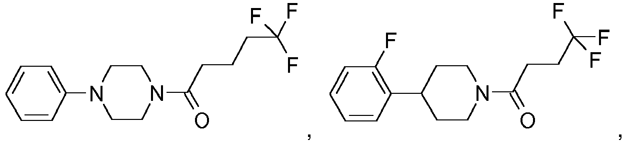 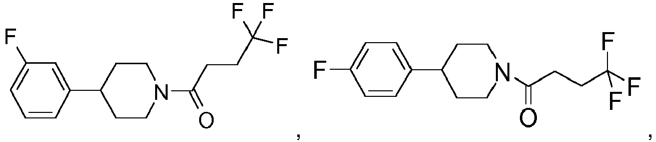 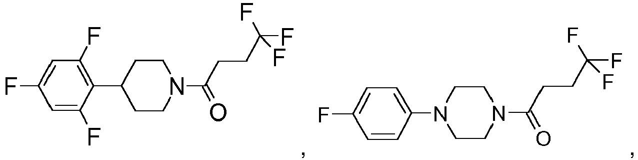 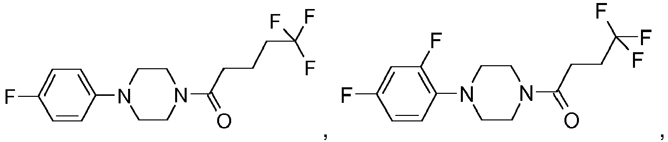 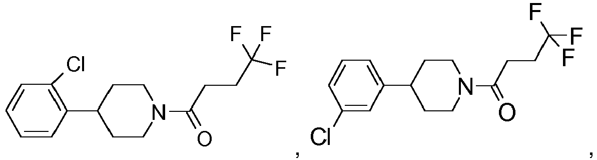 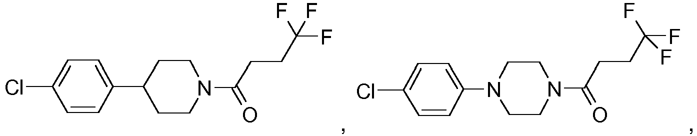 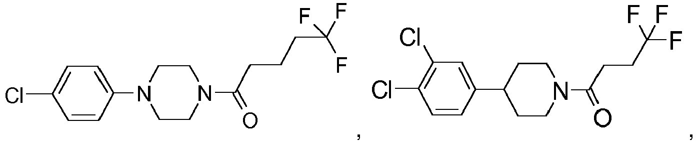 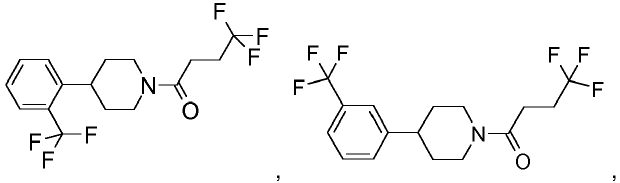 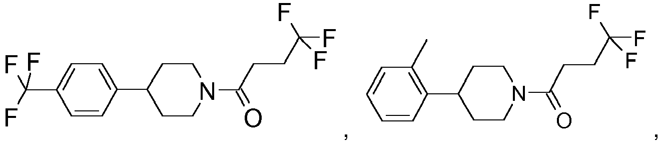 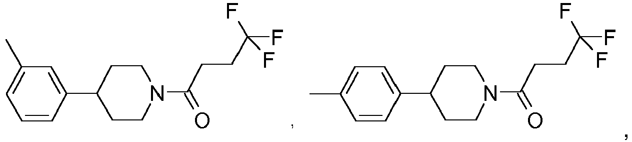 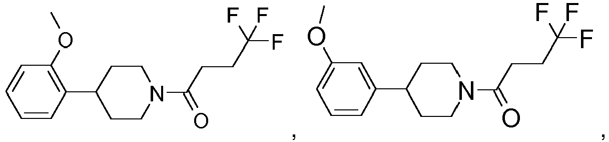 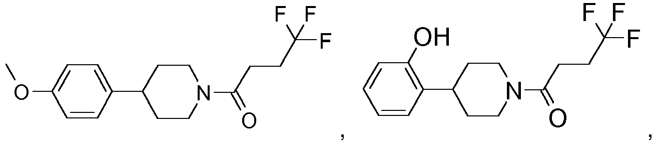 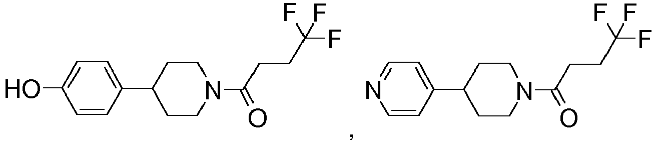 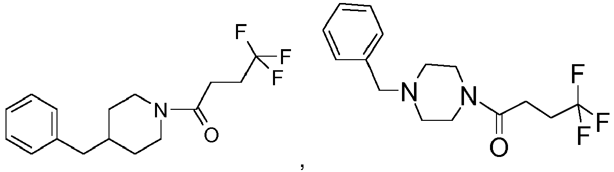 und

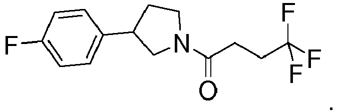

11. Verbindung nach einem der Ansprüche 1 bis 10 zur Verwendung bei der Behandlung von Tuberkulose, Lepra oder atypischen Mykobakteriosen.

12. Pharmazeutische Zusammensetzung, die als Wirkstoff mindestens eine Verbindung nach einem der Ansprüche 1 bis 10 und einen pharmazeutisch unbedenklichen Hilfsstoff umfasst.

13. Pharmazeutische Zusammensetzung, die als Wirkstoff mindestens eine Verbindung nach einem der Ansprüche 1 bis 10 und außerdem als Wirkstoff mindestens ein mithilfe von EthA aktivierbares Antibiotikum umfasst.

14. Pharmazeutische Zusammensetzung nach Anspruch 13, die als Wirkstoff mindestens eine Verbindung nach einem der Ansprüche 1 bis 10 und außerdem als Wirkstoff mindestens ein aus der Familie der Thioamide ausgewähltes Antibiotikum umfasst.

15. Pharmazeutische Zusammensetzung nach Anspruch 14, die als Wirkstoff mindestens eine Verbindung nach einem der Ansprüche 1 bis 10 und außerdem als Wirkstoff Ethionamid oder Prothionamid umfasst.

16. Produkt, das mindestens eine Verbindung nach einem der Ansprüche 1 bis 10 und mindestens ein mithilfe von EthA aktivierbares Antibiotikum, insbesondere ein Antibiotikum, das aus der Familie der Thioamide ausgewählt ist, enthält, als Kombinationsprodukte zur Behandlung von Tuberkulose, Lepra oder mykobakteriellen Infektionen.

## Claims

1. A compound with formula (I): 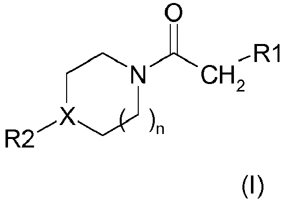 wherein:
n = 0 or 1;
R1 represents a -CH₂CF_{3.} or -CH₂CH₂CF₃ group;
X is chosen from among N and CH;
R2 is chosen from among the following groups:
i. phenyl;
ii. benzyl;
iii. phenyl substituted by at least
a. a linear or branched C1-C4 alkyl chain;
b. a linear or branched C1-C4 alkyl chain and substituted by at least one fluorine atom (F);
c. a group chosen from among Cl, F, CF₃, OCH₃ and OH;
iv. benzyl substituted by at least
a. a linear or branched C1-C4 alkyl chain;
b. a linear or branched C1-C4 alkyl chain and substituted by at least one fluorine atom (F); and
v. 6-membered heterocycles including one, two or three nitrogen atoms.

2. The compound according to claim 1, **characterized in that** n = 1.

3. The compound according to claim 1 or 2, **characterized in that** R1 is a -CH₂CF₃ group.

4. The compound according to any one of the preceding claims, **characterized in that** X = CH.

5. The compound according to any one of claims 1 to 4, **characterized in that** R2 is phenyl or benzyl.

6. The compound according to any one of claims 1 to 4, **characterized in that** R2 is phenyl substituted in the meta position relative to the X bond, by a group chosen from among:
Cl, F, CF₃ and OCH₃.

7. The compound according to any one of claims 1 to 4, **characterized in that** R2 is phenyl substituted by at least one F atom.

8. The compound according to any one of claims 1 to 4, **characterized in that** R2 is phenyl substituted in the para position relative to the X bond, by a fluorine atom F.

9. The compound according to any one of claims 1 to 4, **characterized in that** R2 is a group chosen from among the following groups:

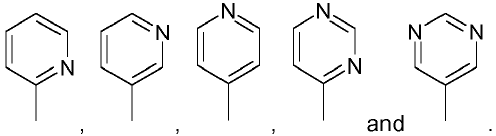

10. The compound according to claim 1, **characterized in that** it is chosen from among the following compounds: 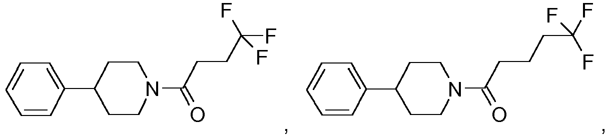 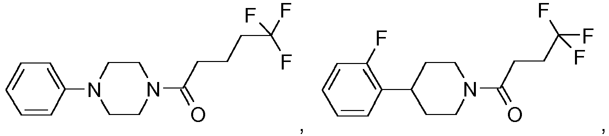 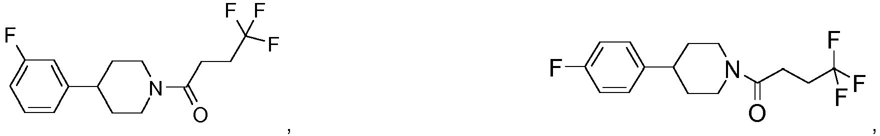 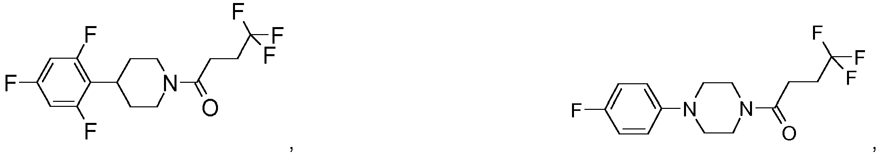 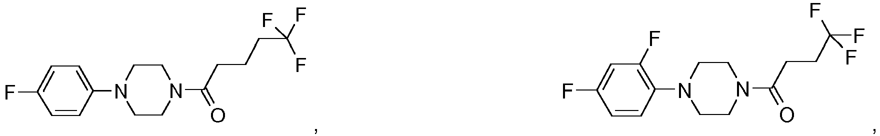 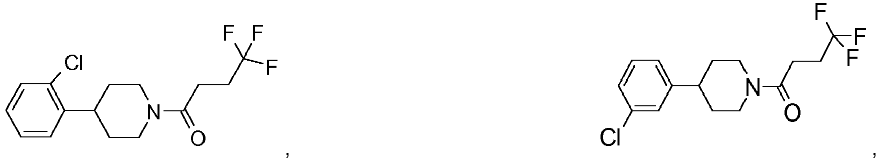 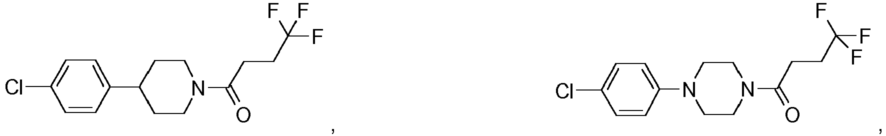 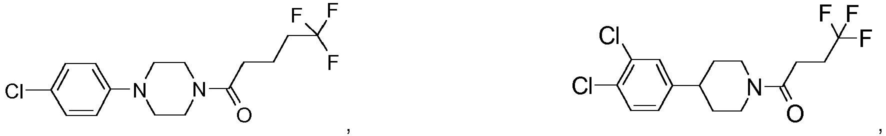 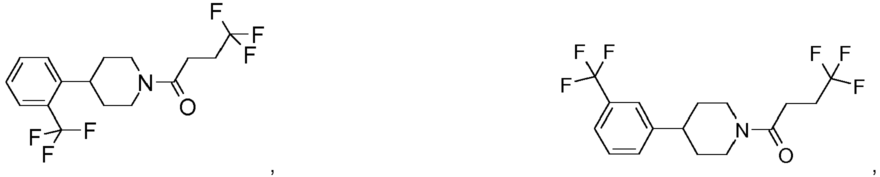 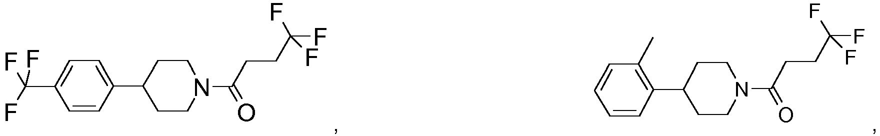 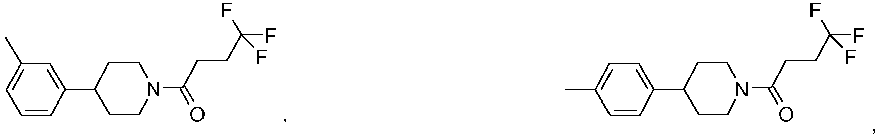 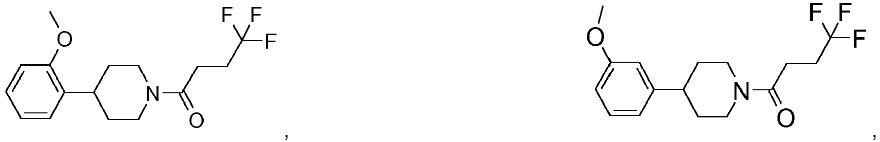 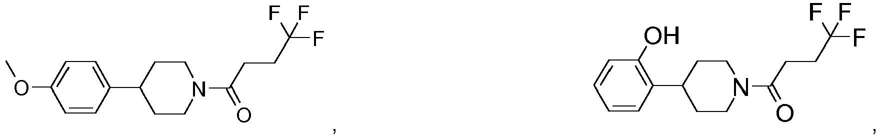 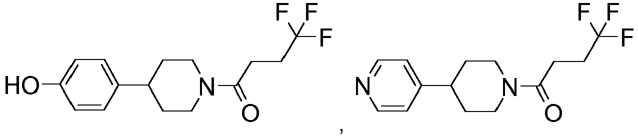 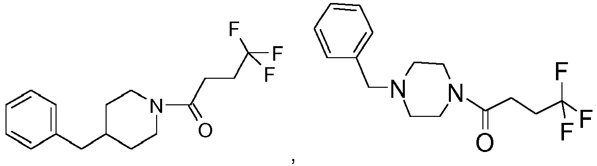 and

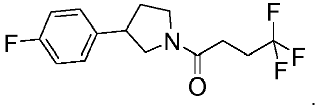

11. The compound according to any one of claims 1 to 10, for use in the treatment of tuberculosis, leprosy or atypical mycobacterioses.

12. A pharmaceutical composition comprising, as active ingredient, at least one compound according to any one of claims 1 to 10 and a pharmaceutically acceptable excipient.

13. A pharmaceutical composition comprising, as active ingredient, at least one compound according to any one of claims 1 to 10, and further, as active ingredient, at least one antibiotic able to be activated through the EthA route.

14. The pharmaceutical composition according to claim 13, comprising, as active ingredient, at least one compound according to any one of claims 1 to 10, and further, as active ingredient, at least one antibiotic chosen from among the family of thioamides.

15. The pharmaceutical composition according to claim 14, comprising, as active ingredient, at least one compound according to any one of claims 1 to 10, and further, as active ingredient, ethionamide or prothionamide.

16. A product containing at least one compound according to any one of claims 1 to 10 and at least one antibiotic able to be activated through the EthA route, more particularly an antibiotic chosen from the family of thioamides, as combination products in tuberculosis, leprosy or general mycobacterial therapy.
